**Europäisches Patentamt**

(19) **European Patent Office**

**Office européen des brevets**

(11) Numéro de publication: **0 005 404**
**B1**

(12) **FASCICULE DE BREVET EUROPEEN**

(45) Date de publication du fascicule de brevet: **30.12.81**

(51) Int. Cl.³: **A 61 K 35/28**

(21) Numéro de dépôt: **79400280.8**

(22) Date de dépôt: **02.05.79**

(54) **Produit cicatrisant extrait de la moelle osseuse d'animaux.**

(30) Priorité: **03.05.78 FR 7813780**
**15.09.78 FR 7827020**

(43) Date de publication de la demande:
**14.11.79 Bulletin 79/23**

(45) Mention de la délivrance du brevet:
**30.12.81 Bulletin 81/52**

(84) Etats Contractants Désignés:
**BE CH DE FR GB IT NL SE**

(56) Documents cités:
**FR - A - 2 257 268**
**FR - M - 4070M**

(73) Titulaire: **Souron, Yves Marie Frank**
**6, Place du Palais**
**F-35000 Rennes (FR)**

(72) Inventeur: **Souron, Yves Marie Frank**
**6, Place du Palais**
**F-35000 Rennes (FR)**

(74) Mandataire: **Le Guen, Louis François**
**13, rue Emile Bara BP 91**
**F-35802 Dinard Cedex (FR)**

Courier Press, Leamington Spa, England.

## Produit cicatrisant extrait de la moelle osseuse d'animaux

La présente invention concerne un nouveau produit a propriétés thérapeutiques extrait de la moelle osseuse d'animaux, et, en particulier, un produit à propriétés cicatrisantes.

On sait que la moelle osseuse renferme trois classes de substances: une classe de substance inhibitrice de la croissance des cellules, une classe de substance accélératrice de la croissance des cellules et une classe de substances oncogènes. Dans le brevet spécial de médicament français 4 070 M, on a développé les propriétés thérapeutiques des substances inhibitrices et on a simplement signalé que les substances accélératrices peuvent être utilisées par exemple en vue de la guérison des plaies externes, sans toutefois préciser de quelles plaies. De plus, on y a considéré simplement l'extraction des trois substances en vue d'obtenir celles-ci sous forme de poudres injectables ou administrées par voie perorale.

Dans le brevet français 2 257 268 déposée par le présent demandeur, il a été indiqué que la moelle entière appliquée sur des plaies avait des propriétés anti-inflammatoires, anti-allergiques et même dans une certaine mesure anti-infectieuses et cicatrisantes. En fait, comme l'indiquaient les essais décrits dans la description de ce brevet, la moelle entière était appliquée sur des parties de peaux non saines, telles que des parties présentant des rougeurs ou des parties brûlées, et, effectivement dans ces cas, le moelle entière avait une action bénéfique. Par contre, sur des plaies franches et saines, il s'est révélé que l'action de la moelle entière était insignifiante, sans doute du fait de l'effet compensateur entre les entre les substances inhibitrices et les substances accélératrices de la croissance des cellules.

Un objet de la présente invention consiste à prévoir un produit à base de substances accélératrices de la croissance des cellules extraites de la moelle osseuse, qui soit directement appliqué sur des plaies saines pour accélérer leur cicatrisation.

Un autre objet de l'invention consiste à prévoir un produit à base des substances accélératrices de la croissance des cellules, extrait de la moelle osseuse, qui soit directement appliqué sur des plaies saines ou même enflammées et infectées, mais sous forme de poudre au lieu de gel.

Suivant une caractéristique de l'invention, il est prévu un produit cicatrisant préparé à partir de la moelle complète, à laquelle on ajoute de l'eau en quantité suffisante, le mélange obtenu étant homogénéisé par action mécanique ou autre, la phase aqueuse obtenue étant additionnée d'éther par exemple, en quantité volumétrique égale, puis agitée pendant environ 30 minutes, avant d'être centrifugée pour obtenir, entre la phase aqueuse et la phase de solvant, les substances que l'on recueille qui forme un gel constituant ledit produit cicatrisant. Il est recommandé d'utiliser une centrifugeuse tournant par exemple à 10.000 tours pour mieux séparer les accélératrices des inhibitrices et accroître ainsi l'efficacité du produit.

Suivant une autre caractéristique de l'invention, ledit gel constituant le produit cicatrisant peut être mélangé avec un produit imperméable à l'air notamment pour des applications sur des plaies enflammées, telles que des plaies eczémateuses.

Suivant une autre caractéristique, le produit imperméable à l'air est en produit gras compatible avec le gel.

Suivant une autre caractéristique, le produit gras est de la graisse de moelle osseuse.

D'après ce qui précède, il apparaît qu'il n'a pas été indispensable de purifier, ni de sécher le produit. Il en résulte que le produit peut être fabriqué facilement d'une manière industrielle et que son coût est nettement plus faible.

Suivant une caractéristique de la présente invention, il est prévu une méthode d'utilisation des substances accélératrices de la croissance des cellules extraites de la moelle consistant à appliquer lesdites substances sous forme de poudre sèche, obtenue par dessiccation du gel, sur les plaies pour cicatriser ces dernières.

Suivant une autre caractéristique, il est prévu, pour l'application, de mélanger la poudre avec du talc ou du stéarate de magnésium, par exemple.

Suivant une autre caractéristique, il est prévu pour l'application de mélanger à la poudre, un corps gras, compatible avec la poudre, pour protéger la plaie d'une oxygénation possible de l'air.

On notera qu'il suffit pour obtenir la poudre d'une simple dessiccation, et non une lyophilisation comme cela devient nécessaire quand on désire obtenir un produit injectable (ou pouvant être administré par voie perorale).

Suivant une autre caractéristique, il est prévu d'ajouter à la poudre, éventuellement déjà additionnée de talc et/ou d'un corps, un produit anti-inflammatoire ou un antibiotique, ou un insecticide pour améliorer encore son action.

Suivant une autre caractéristique, il est prévu une préparation de la poudre formée par les substances accélératrices de la croissance des cellules dans laquelle, à la moelle extraite des os on ajoute de l'eau en quantité appropriée, on homogénéisé le mélange ainsi obtenu, on extrait lesdites substances accélératrices par un solvant, ces opérations étant connues, et on effectue une simple dessiccation desdites substances.

Suivant une autre caractéristique, pour la préparation ci-dessus, on chauffe les os pour en extraite la moelle.

Pour mettre les propriétés cicatrisantes du produit suivant l'invention en évidence, on a procédé à un certain nombre d'expériences dont on va décrire maintenant les modalités d'exécution et les résultats, après avoir précisé plus en détail un exemple de préparation du produit.

Préparation du produit ou gel utilisé
dans les expériences avec gel

On prend de la moelle osseuse fraîche provenant d'os de boeuf. On ajoute à la moelle osseuse de l'eau en quantité suffisante et on homogénéisé le mélange en utilisant un mixeur de manière à séparer la phase aqueuse d'elle-même. On termine cette séparation de la phase aqueuse en utilisant une élévation de pression. Puis, on ajoute à la phase aqueuse restante de l'éther en quantité égale et on agite le mélange pendant 30 minutes. Ensuite, on passe le mélange dans une centrifugeuse à 10.000 tours afin d'obtenir, en suspension entre la phase aqueuse et la phase du solvant, le produit que l'on recueille qui est lavé rapidement pour en éliminer les restes de la phase non soluble dans l'éther. Le produit recueilli a la consistance d'un gel qui constitue un cicatrisant.

On a put vérifier par électrophorèse que ce gel contient des albumines de la classe des substances accélératrices de la croissance des cellules. Il contient également bien entendu de l'éther qui constitue, comme on le sait, un antiseptique.

Modalités d'exécution des expériences
avec gel

Les expériences ont été menées sur des rats WISTAR. On pratique, juste derrière la tête de chaque rat, une incision pour extraire un morceau de peau de 1 cm². En pratique, on enlève toute la peau, y compris l'aponévrose attenante, en laissant l'aponévrose inférieure. Le choix de l'incision derrière la tête s'explique par le souci d'éviter que l'animal ne gratte la croûte croissant sur la plaie et ne fausse ainsi les résultats.

Le produit de l'invention est appliqué tous les jours, ainsi que les autres médications utilisées pour établir des comparaisons.

Premières séries d'expériences avec gel

On utilise 20 rats de 250 g, agés de 8 semaines.

Sur 5 rats, on a appliqué le produit défini ci-dessus et préparé comme indiqué. Sur 5 autres rats, on a appliqué de l'éther éthylique pur. Encore, sur 5 autres rats, on a utilisé de solucire. Enfin, sur les 5 derniers rats, on n'a appliqué aucune médication.

L'application de l'éther pur avait pour but de vérifier si l'éther contenu dans le produit de l'invention n'était pas le seul élément cicatrisant de celui-ci. L'application de solucire avait pour but de vérifier si la cicatrisation provoquée par le produit de l'invention n'était pas due au fait qu'il abrite la plaie de l'action de l'air.

Après deux jours, pour les rats traités par le produit de l'invention, un bourrelet apparaît autour de la plaie soignée, alors que rien ne se produit pour les trois autres séries de rats. Le milieu de la plaie traitée par le produit est durci et il n'y a ni suintement, ni cassure de la plaie. Les plaies traitées à l'éther sont plus grandes, suintantes et cassées par endroits. Les plaies des témoins sont plus grandes que celles traitées par le produit.

Après 8 jours, les plaies traitées par le produit sont guéries, c'est à dire que tout l'épiderme est net et lisse. Les plaies traitées à l'éther ont encore une croûte d'environ 3/4 cm² qui se détache. Les plaies traitées au solucire sont encore très grandes. Les plaies des rats témoins ont sensiblement la même apparence que celles des rats traités à l'éther.

D'une manière plus détaillée pour les plaies traitées par le produit de l'invention, il apparaît au 2ème jour un bourrelet autour de la plaie, ce bourrelet étant constitué par un grossissement de la partie interne des bords de la peau. Au 4ème jour, environ, il se forme une croûte qui se soulève au 7ème jour en laissant apparaître une région très cicatrisée dans la région périphérique autour d'une partie centrale beaucoup plus petite que la surface de l'ancienne croûte, cette partie centrale étant plus rouge, sans que ce rouge soit vif. Cette partie centrale diminue ensuite et laisse seulement une toute petite croûte d'un demi-millimètre carré environ au 8ème jour, ce qui indique pratiquement la fin de la cicatrisation. La région cicatrisée apparaît différente des régions voisines par la petitesse de ses pores qui grossissent jusqu'à la dimension normale après encore 8 à 10 jours. La peau de cette règion est alors lisse et nette, sans aucun kelloïde. Sur les rats traités par le produit, il n'a jamais été constaté de présence de kelloïde. Les poils repoussent de la même couleur que les anciens, très rapidement.

Secondes séries d'expériences avec gel

On utilise encore 20 rats de quelques semaines.

Cinq rats ont été traités avec le produit de l'invention, cinq avec un produit à base de vitamine A connu sous le nom de: "AVIBON", cinq autres avec du Baume du Pérou pur, les cinq derniers rats servent de témoins.

Après 3 jours, pour les rats traités avec le produit, les croûtes sont plus petites; pour les rats traités avec l'Avibon, comme avec le Solucire précédemment, les plaies sont très grandes; pour les rats traités avec le Baume du Pérou, le Baume se refait mais en suintant, l'ouverture de l'épiderme ne paraissant pas s'être réduite; pour les rats témoins, les plaies sont plus grandes qu'avec le produit de l'invention.

Après 7 jours, pour les rats traités par le produit, les croûtes se décollent, toutes font 3/4 cm². Une croûte qui est décollée n'occupe que trois millimètres carrés et le rat correspondant se gratte; pour les rats traités avec l'Avibon, les plaies font toutes environ 1 cm²; il en est de même pour les rats traités avec le Baume du Pérou; pour les rats témoins, les croûtes font 1 cm², mais une est décollée.

Après 8 jours, pour les rats traités par le produit, les croûtes ont toutes disparues, deux rats sont cicatrisés, deux autres le sont pratiquement; pour les rats traités avec l'Avibon, les croûtes ont disparues mais les plaies font encore ½ à 1 cm²; pour les rats traités avec le Baume du Pérou, il n'y a pas d'amélioration, si ce n'est qu'une légère diminution de diamètre; pour les rats témoins, les croûtes ont diminuées, mais font encore de ½ à 1 cm².

Après 9 jours, tous les rats traités par le produit sont guéris.

Après 11 jours, un rat traité avec le Baume du Pérou est guéri.

Après 12 jours, un rat témoin est guéri.

Après 15 jours, un second rat traité avec le Baume du Pérou est guéri, les trois derniers ont encore une croûte de 2 mm². Deux rats traités avec l'Avibon sont guéris, les trois autres ont encore une petite plaie de 2 mm². Les quatre derniers rats témoins ne sont pas encore guéris, trois ont une croûte de 3 mm² et un de 2 mm².

Troisièmes séries d'expériences avec gel

On utilise 9 rats de 30 mois, c'est à dire de vieux rats.

Trois rats ont été traités avec le produit de l'invention, trois avec un produit connu sous le nom de "Madécassol", et enfin, trois rats servent de témoins.

Après quatre jours, les rats traités avec le produit se grattent, mais les croûtes sont tout de même en relief, c'est à dire sur une sorte de promontoire, ce qui ne se produit pas sur les rats traités différemment. Pour les rats traités avec le Madécassol, les croûtes sont beaucoup plus grandes et ne sont pas en relief. Pour les rats témoins les croûtes sont de la même taille que celles des rats traités avec le produit, mais ne sont pas en relief.

Après 8 jours, pour les rats traités avec le produit, 3 croûtes existent encore mais sont décollées au trois quarts, chaque croûte est très épaisse, beaucoup plus épaisse que sur de jeunes rats. Pour les rats traités avec le Madécassol, les trois croûtes sont de même taille que celles des rats traités avec le produit, mais ne sont pas décollées; sous les croûtes les plaies sont suintantes; les plaies sont aussi grandes que les croûtes alors que pour les plaies traitées avec le produit, la cicatrisation a rendu les plaies restantes plus petites que les croûtes. Pour les rats témoins, les trois croûtes sont à vif et complètement collées.

Après 10 jours, les trois rats traités par le produit n'ont plus du tout de croûte et leurs plaies sont cicatrisées. Pour les rats traités par le Madécassol, un rat a sa croûte décollée, les autres non. Pour les rats témoins, une croûte est décollée, mais la plaie n'est pas guérie, et les deux autres croûtes sont collées.

Après 12 jours, un rat traité au Madécassol est guéri, les témoins ne sont pas encore guéris.

Après 16 jours, les deux derniers rats traités au Madécassol ont encore des croûtes de 2 mm²; aucun des témoins n'est guéri, leurs croûtes font de 3 à 2 mm².

Conclusions des expériences avec gel

Des résultats d'expériences mentionnés ci-dessus, il apparaît que le produit suivant l'invention a des propriétés cicatrisantes nettement améliorées par rapport à celles des meilleurs cicatrisants communément utilisés.

Il faut noter également que l'éther, outre les faits qu'il soit antiseptique et utilisé dans la fabrication comme solvant, joue également un certain rôle dans la conservation du produit.

Quand on utilise un autre solvant que l'éther pour extraire le produit, et même en utilisant de l'éther, il peut être intéressant d'ajouter au produit un antiinflammatoire et un conservateur. Par ailleurs le solvant utilisé doit être de préférence antiseptique.

Enfin, il est également possible de recueillir les substances constituant le produit et se trouvant après l'opération de centrifugation entre la phase aqueuse et la phase du solvant sous la forme d'un disque, en aspirant ces substances au moyen d'une pipette dont l'entrée est plongée dans le disque. Ensuite on lave le produit à l'eau distillée en l'agitant.

Préparation du produit en poudre utilisé dans les expériences avec poudre

On part du gel préparé comme indiqué ci-dessus et on procède simplement par déshydratation de ce gel jusqu'à obtenir une poudre qui constitue le nouveau produit cicatrisant.

Modalités des expériences avec poudre

Les expériences ont été menées sur des rats WISTAR. On pratique, juste derrière la tête de chaque rat, une incision pour extraire un morceau de peau de 1 cm². En pratique, on enlève toute la peau, y compris l'aponévrose attenante, en laissant l'aponévrose inférieure.

Le produit est appliqué tous les jours, ainsi que les autres médications utilisées pour établir des comparaisons.

Le produit expérimenté est de la poudre extraite de la moelle, comme on l'a indiqué ci-dessus, mélangé avec du talc pur, dans les proportions suivantes: 0,7 g de poudre pour 27 g de talc.

On aurait pu utiliser de la poudre seule, mais, en réalité, il suffit de très peu de produit sec pour obtenir l'effet cicatrisant avec une bonne efficacité, la quantité étant trop faible pour déssécher la plaie. Par contre, en ajoutant du talc, comme excipient, on obtient cet effet de déssechement de la plaie.

Premières séries d'expériences avec poudre

On utilise 20 rats de 250 g, âgés de 8 mois.

Sur cinq rats, on a appliqué le mélange poudre active et talc que l'on a défini ci-dessus. Sur cinq autres rats, on a appliqué un gel formé également des substances accélératrices de la croissance des cellules, tel qu'il a été défini dans la demande de brevet N°: 78 13780 déjà mentionnée, et que l'on retrouve dans la préparation mentionnée ci-dessus avant l'opération de dessiccation. Encore sur cinq rats, on a appliqué uniquement du talc. Enfin, sur les cinq derniers rats on n'a appliqué aucune médication.

Après deux jours, pour les rats traités avec le produit en poudre, les plaies sont nettement désséchées et plus petites que pour les autres rats. Pour les rats traités au gel, les plaies sont un peu moins grandes que celles des témoins. Pour les rats traités au talc, les plaies sont identiques à celles des témoins.

Après quatre jours, pour les rats traités avec le produit en poudre, les plaies sont nettement plus petites que celles des autres rats. Pour les plaies traitées avec le gel, elles ont également diminuées, mais moins vite. Pour les rats traités au talc et les rats témoins, les plaies sont à peine diminué depuis l'incision et présentent un peu de sérosité.

Après sept jours, les plaies des rats traités par le produit en poudre sont complètement guéries.

Après huit jours, les plaies des rats traités par le gel sont complètement guéries.

Ce n'est qu'après quatorze jours que les plaies traitées au talc et celles qui n'ont reçu aucune médication sont guéries.

Secondes séries d'expériences avec poudre

Au cours des expériences ci-dessus, il est apparu que certains rats témoins avaient leurs plaies qui s'infectaient. Pour réduire ces plaies, on y a appliqué le produit en poudre et on a constaté que les plaies infectées guérissaient.

A noter encore qu'aucune des plaies traitées avec la poudre ne s'est infectée, aucune des plaies traitées avec le gel ne s'est non plus infectée.

On peut donc en conclure que le produit formé par les substances accélératrices de la croissance des cellules, que l'on extrait de la moelle, ont des propriétés anti-infectieuses, cette poudre ne contenant plus d'éther ni aucun additif sauf le talc.

Il s'agit là d'une découverte car, si on savait qu'une albumine contenue dans le sang avait des propriétés anti-infectieuses, cela n'avait pas encore été mis en évidence pour des albumines de la moelle.

Troisièmes séries d'expériences avec poudre

Dans cette série d'expériences, on utilise 20 rats âgés de 36 mois, c'est à dire des rats très âgés.

Sur cinq rats, on appliqué le mélange poudre active et talc, déjà défini ci-dessus. Sur cinq autres rats, on a utilisé du gel, également déjà défini. Sur cinq autres rats, on a utilisé du talc seul. Les cinq derniers rats servent de témoins.

Les résultats one été les suivants: les rats traités à la poudre ont leurs plaies guéries en 10 jours. Ceux qui ont été traités au gel ont leurs plaies guéries en 14 jours, tandis que les rats traités au talc et les rats témoins ont leurs plaies qui s'infectent.

Il faut noter que ces expériences sur des plaies de rats âgés revêtent une particulière importance, car les plaies de vieux rats représentent un modèle expérimental des escarres.

A noter encore que les plaies infectées des rats traités au talc et des témoins ont toutes été guéries par la suite après application de la poudre active extraite de la moelle, ce qui met encore en évidence les propriétés anti-infectieuses de cette poudre.

Conclusions des expériences avec poudre

De ce qui précède, il résulte que l'utilisation desdites substances accélératrices de la croissance des cellules sous forme de poudre, seule ou mélangée avec du talc ou un produit équivalent, permet de cicatriser très rapidement des plaies en y évitant toute infection.

Il résulte également que les substances accélératrices de la croissance des cellules, qui sont tirées de la moelle osseuse, contiennent un composant anti-infectieux ou mieux anti-bactérien et que donc ces substances peuvent être utilisées comme tel, notamment sous forme de poudre.

Il faut encore noter que, si l'efficacité de la poudre par rapport à celle du gel est plus grande pour les jeunes rats, l'amélioration apportée par la poudre par rapport au gel est encore plus sensible sur les vieux rats.

En plus du talc, on peut ajouter à la poudre un corps gras approprié dont la fonction est alors de maintenir la plaie à l'abri de l'air. Toutefois, certains corps gras sont à proscrire, tels que la vaseline ou le solucire. On a, en effet, noté que la poudre se diffuse mal dans la vaseline, ce qui peut empêcher d'atteindre des portions de plaie à traiter. On peut aussi ajouter à la poudre un produit anti-inflammatoire. On peut également aider à la conservation de la poudre en lui ajoutant un produit conservateur approprié si nécessaire.

Il doit être bien entendu que l'on n'a décrit une méthode de préparation de la poudre qu'à titre d'exemple, mais que d'autres méthodes d'extraction sont également utilisables, dont notamment celles qui sont exposées dans le brevet français 4 070 M. La préparation peut être effectuée en utilisant une séquence de chauffage, et d'autres solvants adéquats.

Autres expériences menées avec le produit en poudre

On a pris le produit sec en poudre et on l'a rendu injectable par un procédé classique. On a injecté ce produit dans le coussin plantaire de la patte d'un rat. Puis on a injecté au même endroit, dans un premier essai, de la caroghénine et, dans un second essai, du kaolin, chacun de ces produits devant habituellement provoquer une forte enflure de la patte. Or, après les essais, on a constaté que l'enflure est à peine apparente et, en tous cas, très inférieure à celle que l'on constate habituellement. L'extrait de moelle, suivant l'invention a donc bien une action contre les effets de la caroghénine ou du kaolin.

Modalités d'exécution de ces dernières expériences
*Essais contre l'effet de la caroghénine*

L'extrait de molle injectable, c'est à dire auquel on a ajouté de l'eau distillée est injecté dans le coussin plantaire de 10 rats Wistar de 9 mois.

Aussitôt après cette injection, on fait, à chacun des rats, une piqûre pour leur injecter dans la patte 0,10 mg du mélange suivant: sérum physiologique, plus 2% de caroghénine.

En l'absence de la piqûre préventive d'extrait de moelle, le mélange sérum + caroghénine provoque habituellement une très forte enflure de la patte piquée.

Dans les essais indiqués, on a constaté que l'enflure était soit très faible, soit pratiquement inexistante.

Essais contre l'effet du kaolin

L'extrait de moelle injectable est injecté dans le coussin plantaire de 10 autres rats Wistar agés de 9 mois.

Aussitôt après l'injection, on fait, à chacun des rats, une piqûre pour leur injecter dans la patte 0,10 mg du mélange suivant: sérum physiologique, plus 10% de kaolin.

Au lieu de l'enflure caractéristique habituelle, on a constaté que pour chaque rat l'enflure était soit très faible, soit inexistante, ce qui est essentiellement dû à l'effet préventif de l'extrait de moelle injecté.

Autres conclusions

Il est bien évident que les effets des extraits de moelle qui ont été décrits ci-dessus, à propos du produit en gel ou en poudre, sont encore valables quand on rend ce produit injectable.

Revendications

1. Produit cicatrisant comportant des substances accélératrices de la croissance des cellules, ces substances existant naturellement dans la moelle osseuse, le produit étant préparé à partir de moelle osseuse complète à laquelle on ajoute de l'eau en quantité convenable, le mélange obtenu étant homogénéisé par action mécanique, puis la phase aqueuse du mélange homogénéisé étant séparée en utilisant une élévation de pression, puis additionnée d'un solvant antiseptique tel que l'éther en quantité volumétrique égale, puis agitée pendant environ 30 minutes, caractérisé en ce que le mélange agité est ensuite centrifugé pour obtenir, entre la phase aqueuse et la phase de solvant, un gel qui constitue le produit cicatrisant.

2. Produit cicatrisant suivant la revendication 1, caractérisé en ce que ledit gel est mélangé avec un produit imperméable à l'air.

3. Produit cicatrisant suivant la revendication 2, caractérisé en ce que ledit produit imperméable à l'air est un produit gras compatible avec le gel.

4. Produit cicatrisant suivant la revendication 3, caractérisé en ce que ledit produit gras est de la graisse de moelle osseuse.

5. Produit cicatrisant suivant la revendication 1, caractérisé en ce que le gel est desséché pour obtenir une poudre sèche.

6. Produit cicatrisant suivant la revendication 5, caractérisé en ce que ladite poudre est mélangée avec du talc.

7. Produit cicatrisant suivant la revendication 5 ou 6, caractérisé en ce que ladite poudre est mélangée avec un corps gras compatible.

8. Produit cicatrisant suivant l'une des revendications 1 à 7, caractérisé en ce que la moelle osseuse initiale est de la moelle d'os de bovin.

Patentansprüche

1. Mittel zur Förderung der Vernarbung, das Substanzen zur Beschleunigung des Zellwachstums enthält, welche Substanzen ihrer Natur nach aus Knochenmark bestehen, wobei das Mittel erzeugt wird aus vollständigem Knochenmark, dem Wasser in geeigneter Menge zugegeben wird, worauf die erhaltene Mischung mechanisch homogenisiert wird, wonach die wässrige Phase der homogenisierten Mischung mittels Druckerhöhung abgetrennt und dann mit einem antiseptischen Lösungsmittel, wie z.B. Äther, in gleicher Volumen-Menge gemischt und dann etwa 30 Minuten lang gerührt wird, dadurch gekennzeichnet, daß die durchgerührte Mischung dann zentrifugiert wird, um zwischen der wässrigen Phase und der Lösungsmittel-Phase ein Gel zu erzeugen, welches das die Vernarbung fördernde Mittel bildet.

2. Mittel nach Anspruch 1, dadurch gekennzeichnet, daß das Gel mit einer luftundurchlässigen Substanz gemischt wird.

3. Mittel nach Anspruch 2, dadurch gekennzeichnet, daß die luftundurchlässige Substanz eine Fett-Substanz ist, die mit dem Gel verträglich ist.

4. Mittel nach Anspruch 3, dadurch gekennzeichnet, daß die Fett-Substanz Fett aus Knochenmark ist.

5. Mittel nach Anspruch 1, dadurch gekennzeichnet, daß das Gel getrocknet wird, um ein trockenes Pulver zu erzeugen.

6. Mittel nach Anspruch 5, dadurch gekennzeichnet, daß das Pulver mit Talk gemischt wird.

7. Mittel nach Anspruch 5 oder 6, dadurch gekennzeichnet, daß das Pulver mit einer verträglichen Fett-Substanz gemischt wird.

8. Mittel nach einem der Ansprüche 1 bis 7, dadurch gekennzeichnet, daß das ursprüngliche Knochenmark solches aus Rinderknochen ist.

## Claims

1. A cicatrizing product including substances increasing cell growth, those substances existing by nature in bone marrow, the product being prepared from complete bone marrow to which water is added in proper quantity, the produced mixture being homogenized through mechanical action, then the aqueous phase of the homogenized mixture being separated by using pressure increase, then mixed with an antiseptic solvent such as ether in equal volumetric quantity, then stirred for about 30 minutes, characterized by the fact that the stirred mixture is then centrifuged for producing between the aqueous phase and the solvent phase a gel that constitutes the cicatrizing product.

2. A cicatrizing product according to claim 1, characterized by the fact that the said gel is mixed with an air impervious product.

3. A cicatrizing product according to claim 2, characterized by the fact the said air impervious product is a fatty product compatible with the gel.

4. A cicatrizing product according to claim 3, characterized by the fact that the said fatty product is grease from bone marrow.

5. A cicatrizing product according to claim 1, characterized by the fact that the gel is dried to produce a dry powder.

6. A cicatrizing product according to claim 5, characterized by the fact that the said powder is mixed with talc.

7. A cicatrizing product according either to claim 5 or 6, characterized by the fact that the said powder is mixed with a compatible fatty product.

8. A cicatrizing product according to any one of claims 1—7, characterized by the fact that the initial bone marrow is marrow from bovine bones.